# EUROPÄISCHE PATENTSCHRIFT

(11) **EP 2 087 487 B1**
(45) Veröffentlichungstag und Bekanntmachung des Hinweises auf die Patenterteilung: **28.09.2016**
(21) Anmeldenummer: 07819130.1
(22) Anmeldetag: 19.10.2007
(51) Int. Cl.: G11B 7/00

(54) **SUBSTRATMATERIALIEN FÜR TRANSPARENTE SPRITZGUSSKÖRPER**
SUBSTRATE MATERIALS FOR TRANSPARENT INJECTION MOULDINGS
MATÉRIAUX DE SUBSTRAT POUR CORPS TRANSPARENTS MOULÉS PAR INJECTION

(30) Priorität: 31.10.2006 DE 102006051309
(43) Veröffentlichungstag der Anmeldung: 12.08.2009
(73) Patentinhaber: Covestro Deutschland AG, 51373 Leverkusen (DE)
(72) Erfinder: MEYER, Alexander, 40489 Düsseldorf (DE); EBERT, Wolfgang, 47800 Krefeld (DE); HAESE, Wilfried, 51519 Odenthal (DE); KRETSCHMAR, Gabriele, 47829 Krefeld (DE); REITZE, Burkhard, 51429 Bergisch Gladbach (DE); WINZEN, Thomas, 47239 Duisburg (DE); BRUDER, Friedrich-Karl, 47802 Krefeld (DE); RUYTINX, Bert, 3545 Halen (BE); KARBACH, Alexander, 47800 Krefeld (DE); VOETZ, Matthias, 51375 Leverkusen (DE); POGODA, Andreas, 47475 Kamp-Lintfort (DE)
(74) Vertreter: Levpat
(86) Internationale Anmeldenummer: PCT/EP2007/009065
(87) Internationale Veröffentlichungsnummer: WO 2008/052669

(56) Entgegenhaltungen:
- EP-A- 1 285 940
- WO-A-01/62867
- WO-A-2004/028682
- DE-A1- 3 117 611
- DATABASE WPI Week 200529 Derwent Publications Ltd., London, GB; AN 2005-276306 XP002479189 & JP 2005 082647 A (TEIJIN KASEI LTD) 31. März 2005 (2005-03-31)

## Beschreibung

Gegenstand der Erfindung sind Substratmaterialien, die zur Herstellung transparenter Spritzgusskörper, insbesondere zur Herstellung von Spritzgusskörpern, die für die Herstellung von Datenträgem, welche mit so genannten blauen Lasern ausgelesen werden geeignet sind, sowie weitere Formteile aus den erfindungsgemäßen Materialien.

Bei den transparenten Spritzgusskörpern kann es sich z. B. um transparente Platten, Linsen, optische Speichermedien, oder auch um Artikel aus dem Bereich des Automotive Glazings wie z. B. Streulichtscheiben handeln, bei denen eine hohe optische Qualität nötig ist.

Transparente Spritzgusskörper sind vor allem im Bereich des Glazings und der Speichermedien von Bedeutung.

Optische Datenaufzeichnungsmaterialien sind in der letzten Zeit in wachsendem Maße als ein variables Aufzeichnungs- und/oder Archivierungsmedium für große Datenmengen in Gebrauch gekommen. Beispiele für diese Art von optischen Datenspeichern sind z. B. CD, Super- Audio-CD, CD -R, CD -RW, DVD, DVD - R, DVD+R, DVD - RW, DVD+RW und BD.

Für optische Speichermedien werden typischerweise transparente thermoplastische Kunststoffe eingesetzt wie zum Beispiel Polycarbonat, Polymethylmethacrylat und chemische Modifikationen hiervon. Als Substratmaterial insbesondere für einmal beschreibbare und mehrfach auslesbare sowie auch für mehrfach beschreibbare optische Disks eignet sich vor allem aromatisches Polycarbonat, insbesondere Polycarbonat auf Basis von Bisphenol A. Polycarbonat eignet sich ebenfalls gut für die Herstellung von Formteilen aus dem Bereich des Automotive Glazings, wie z. B. von Streulichtscheiben. Dieser thermoplastische Kunststoff verfügt über eine ausgezeichnete mechanische Stabilität, ist wenig anfällig gegenüber Dimensionsveränderungen und zeichnet sich durch eine hohe Transparenz und Schlagzähigkeit aus.

Um größere Datenmengen auf einem Datenträger zu speichern, wurden nach der CD Formate wie die DVD entwickelt. Diese erlauben aufgrund ihrer erhöhten Speicherkapazität z. B. das Speichern von Spielfilmen. So besitzt die DVD im Vergleich zur CD eine 6- bis 8-fach größere Aufnahmekapazität. Dies wurde möglich durch eine Verringerung der Laserwellenlänge von ca. 780 nm auf ca. 655 nm beim DVD Format. Des Weiteren wurde die numerische Apertur des optischen Auslesesystems erhöht.

Die Entwicklung neuer Formate wie z. B. die HD-DVD (High Density DVD) oder die so genannte Blu ray Disk (BD) arbeiten wiederum mit kürzeren Laserwellenlängen. Bei der BD wird durch ein transparentes Coverlayermaterial (PC-Film) ausgelesen.

Dadurch werden die Anforderungen an das durchleuchtete Substratmaterial oder das durchleuchtete Coverlayermaterial immer höher. Je höher die Speicherdichte des jeweiligen Mediums, desto höher sind die qualitativen Anforderungen (z. B. geringer Partikelgehalt) an das Substrat-material. Fehlstellen in der durchleuchteten Substratschicht bzw. in dem durchleuchteten Coverlayermaterial führen zu Fehlern im Ausleseprozess.

So sind insbesondere Fehlstellen, welche mit dem Laserstrahl des Auslesesystems in Wechselwirkung treten können, von besonderer Relevanz hinsichtlich eines fehlerfreien Ausleseprozesses. Dazu gehören bekanntermaßen Fremdpartikel, wie z. B. Staubpartikel oder Metallpartikel, die den Laserstrahl absorbieren und/oder streuen können. Durch die Verringerung der Wellenlänge des Ausleselasers sind zusätzlich solche Partikel schädlich, deren Absorption innerhalb der Wellenlänge des verwendeten Lasers liegt. Bei der HD-DVD (High Density DVD) oder Blu ray Disk sind dies z. B. Wellenlängen zwischen 400 nm - 410 nm.

Aufgrund ihres Absorptionsverhaltens zeigen diese Partikel bei Bestrahlung unter UV-Licht eine charakteristische, störende Fluoreszenz.

Es ist bekannt, dass Substratmaterialien für optische Datenspeicher Fremdkörper enthalten. In EP-A 379130 ist z. B. beschrieben, dass diese Fremdkörper das Beschreiben oder das Auslesen eines optischen Datenträgers mittels eines Laserstrahls negativ beeinflussen können. Ein Substratmaterial besitzt dann eine gute Qualität, wenn ein bestimmter, in diesem Patent beschriebener, Partikelgehalt (Partikelindex) unterhalb eines bestimmten Werts liegt. Bei den Fremdkörpern kann es sich um Staub oder um verkohltes Material handeln. Diese vom Substratmaterial deutlich zu unterscheidenden Fremdkörper werden in einem Lösungsmittel detektiert, in welchem das Substratmaterial selbst löslich ist. Die Fremdkörper die in diesem Lösungsmittel detektiert werden können, sind in der vorliegenden Erfindung nicht gemeint. Die hier beschriebenen Fremdpartikel sind in einem Lösungsmittel, in welchem das Substratmaterial löslich ist, nicht zu detektieren, da sie einen ähnlichen Brechungsindex wie Polycarbonat besitzen. So sind diese Partikel beispielsweise durch einen dem Stand der Technik entsprechenden Partikeltest nach HyacRoyco, wie er z. B. in DE 10248951 beschrieben ist, nicht zu detektieren.

In JP-A-02-135222 werden Fremdkörper mit Gel-artigem Charakter beschrieben, welche einen von der Matrix verschiedenen Brechungsindex aufweisen. Diese Partikel können ebenfalls die Qualität des optischen Datenspeichers mindern. Diese Partikel lassen sich z. B. durch Zusatz von Wasser in einem Extrusionsprozess deutlich verringern. Diese Partikel unterscheiden sich von den hier beschriebenen Partikeln, da der Brechungsindex der hier beschriebenen Partikel im Wesentlichen nicht von dem der Matrix abweicht. Ferner lassen sich die hier beschriebenen Partikel nicht durch den Zusatz von Wasser in einem Extrusionsprozess entfernen bzw. deren Gehalt reduzieren. Auch wird in JP-A 02-135222 nur die Anzahl der Partikel als relevant angesehen. Es wird unterstellt, dass alle Teilchen die gleiche Größe aufweisen, bzw. dass alle Teilchen zur selben Schädigung führen.

In US 6723824 B1 werden Fremdpartikel in Polycarbonat, hergestellt nach dem Schmelzeumesterungsverfahren beschrieben, die bei Bestrahlung mit Licht einer Wellenlänge von 380 nm fluoreszieren und eine Partikelgröße von 30 µm oder größer aufweisen und welche nach einem Alterungstest zu "white spots" in einem Spritzgusskörper führen.

Demgegenüber betrifft die hier beschriebene Erfindung auch Fremdkörper, welche z. T. deutlich kleiner als 30 µm sind, ein anderes Viskositätsverhalten als das Substratmaterial aufweisen und zu Fließstörungen im resultierenden Spritzgusskörper führen können.

In JP2001-310935 wird ein Polycarbonat mit transparenten Fremdkörpern beschrieben. Diese Fremdkörper unterscheiden sich von der Polymermatrix im Brechungsindex. Über die fluoreszierenden Eigenschaften dieser Partikel wird nichts ausgesagt. Eine Größenverteilung wird nicht beschrieben.

JP 2005 082647 A beschreibt eine Mischung, also ein Blend, aus Polycarbonat und 0,01 - 100 Gewichtsteile fluoreszierendem Material. Das Material, was dem Polycarbonat zugesetzt wird, enthält 40 Gew.% oder weniger Teilchen mit einem Partikeldurchmesser < 10 µm und 10 Gew.% oder weniger der Teilchen haben einen Durchmesser > 50 µm. Die in der vorliegenden Anmeldung beanspruchte Teilchengröße ist daher nicht offenbart.

In WO 2004/028682 wird die räumliche Verschlüsselung einer Polymormatrix in Form von Kügelchen oder Körnchen beschrieben, zum Einsatz in u.a. der kombinatorischen Festsphasensynthese. Dieses Dokument betrifft ein von der vorliegenden Erfindung verschiedenes Anwendungsgebiet.

WO 2001/62867 A werden Mischungen aus fluoreszierenden Pigmenten (z.B. Farbstoffe), die einer polymeren Matrix zugesetzt werden, beschrieben. Es handelt sich hierbei nicht µm ein Substratmaterial, das herstellungsbedingt bestimmte fluoreszierende Partikel enthält.

DE 31 17 611 A beschreibt eine Methode zur Messung fluoreszierender Latexpartikel, bei denen eine Fritte aus Polycarbonat zur Abtrennung fluorochromierter Antikörper eines Fluoroimmuno-Assays auf Latexpartikel eingesetzt wird. Der Gegenstand ist daher verschieden vom Gegenstand der vorliegenden Anmeldurg.

EP 1 285 940 beschreibt ein optisches Datenspeichersubstrat aus Polycarbonat, bei dem der Gehalt von ungelösten Substanzen, die Licht bei einer Wellenlänge von 380 Nanometer ausstrahlen, eine Größe von 30 µm oder größer haben, 100 Teile oder weniger pro Kilo Resin ist. Die in der vorliegenden Anmeldung beanspruchte Teilchengrößenverteilung wird nicht beschrieben.

Es wurde nun überraschenderweise festgestellt, dass die Größe der einzelnen Teilchen, die bei einer Anregungswellenlänge von 400-440 nm Fluoreszenzlicht ausstrahlen, unterschiedlich ist und sich diese Teilchen aufgrund ihres viskosen Verhaltens in mehrere Teilchen zerteilen lassen wie z. B. durch einem Extrusionsprozess. Eine sehr viel genauere Aussage über die Qualität des Substratmaterials ist durch die Auswertung der Größenverteilung der fluoreszierenden Fehlstellen möglich.

Es wurde gefunden, dass Substratmaterialien, insbesondere Substratmaterialien auf Polycarbonatbasis, Partikel enthalten, die bei einer Anregungswellenlänge von 400-440 nm Fluoreszenzlicht ausstrahlen, wobei die Wellenlänge innerhalb des oben beschriebenen kritischen Absorptionsspektrums eines blauen Lasers liegt. Überraschenderweise wurde gefunden, dass ein Großteil dieser fluoreszierenden Partikel andere physikalische Eigenschaften als die Polymermatrix (Polycarbonat) selbst aufweisen. Diese im Substratmaterial befindlichen Defektstellen können im fertigen Spritzgussteil, wie zum Beispiel einer optischen Scheibe, zu Fließstörungen führen. Somit unterscheiden sich diese Partikel in ihrem physikalischen Verhalten von der Polymermatrix. Die fluoreszierenden Partikel im Granulat bzw. die fluoreszierenden Fließstörungen im Spritzgusskörper zeichnen sich durch folgende Eigenschaften aus:
Messungen der mechanischen Eigenschaften der Teilchen ergeben einen höheren Modul sowie eine größere Härte im Vergleich zum Matrixmaterial (Polycarbonat). Die Härte der Teilchen liegt dabei um bis zu 0.3 GPa höher als die der Matrix. Die Härtewerte wurden mit einem Nanoindenter der Firma Hysitron an einer Diskoberfläche (Teilchen vs. Matrix) bestimmt.

Je nach Lage der fluoreszierenden Partikel im Spritzgusskörper werden Fließstörungen und somit Defekte an der Oberfläche des Spritzgusskörpers ausgelöst. Diese Fließstörungen, welche selbst fluoreszieren, sind nicht nur für optische Datenspeicher, welche mit einem blauen Laser durchstrahlt werden, relevant, sondern auch für andere Spritzgusskörper, wie z. B. Linsen oder Streuscheiben, da diese Fließstörungen generell die optische Qualität mindern. Bei optischen Datenspeichern werden diese Fließstörungen im Produktionsprozess von einem Scanner detektiert. Die fehlerhaften Disks werden aussortiert.

Der optische Defekt auf einem Spritzgusskörper kann im Falle einer Disk eine Länge von mehreren Millimetern erreichen und ist radial orientiert. Innerhalb dieses optischen Defektes befinde sich eine fluoreszierende Fließstörung. Es wurde gefunden, dass die fluoreszierenden Fließstörungen z. B. an Disks ein Längen/Breiten-Verhältnis von 2 - 30 im Mittel von 5 - 15 aufweisen. Die Länge der fluoreszierenden Fließstörungen liegt zwischen 10 und 200 µm. Diese Werte sind stark abhängig von den eingestellten Maschinenparametern beim Spritzgussprozess, vor allem der Zykluszeit.

Es wurde ferner gefunden, dass die fluoreszierenden Körper ein Relaxationsverhalten aufweisen. Nach dem diese fluoreszierenden Fließstörungen für 2 Minuten bei 300°C getempert wurden, findet man ein Längen/Breiten-Verhältnis von ca. 1, d.h. die längliche fluoreszierende Fließstörung relaxiert zu einem sphärischen Körper.

Die fluoreszierenden Fließstörungen zeigen ein bestimmtes Quellverhalten. Versetzt man diese Fließstörung für 5 Minuten mit Dichlormethan, so erhält man eine Zunahme der Fläche des fluoreszierenden Bereiches. Daraus wurde die Volumenzunahme des Defektes bestimmt - es ergibt sich eine Zunahme um den Faktor 1.1 bis 3.5 (Das Kugelvolumen des Defektes wurde aus dem Radius des flächengleichen Kreises bestimmt). Im Mittel erhält man eine Zunahme um den Faktor 2. Die Polymermatrix selbst löst sich im Falle von Polycarbonat vollständig in Dichlormethan auf, wohingegen der fluoreszierende Körper ungelöst bleibt.

Es wurde ferner gefunden, dass die fluoreszierenden Partikel bestimmte Farbwerte annehmen. Dazu wurden die Partikel mit Licht einer Quecksilberdampflampe durch ein Stufenfilter mit Durchgang ab 470 nm bestrahlt. Die Farbe der Partikel wurde mit einer digitalen Farbkamera Axiocam HRc der Firma Zeiss eingebaut in einem Mikroskop Axioplan 2 der Firma Zeiss nach dem HSI (Hue, Saturation, Intensity)-Farbmodell bestimmt. Die Methode ist z. B. beschrieben in "Digitale Bildverarbeitung mit dem PC", Hans-Jürgen Schlicht, Addison-Wesley, 1993. Misst man die Farbe der fluoreszierenden Partikel, so findet man einen Huewert im Mittel von ca. 80° (von minimal 20° bis maximal 180°), eine Farbsättigung im Mittel von 150 Digits (minimal 10 bis maximal 190) und eine Farbintensität im Mittel von 190 Digits (von minimal 50 bis maximal 255). Die Farbe der Matrix liegt im Falle von Polycarbonat (Compact Disk) bei einen Huewert von ca. 75° (von minimal 74° bis maximal 78°), eine Farbsättigung im Mittel von 133 Digits (minimal 132 bis maximal 134) und eine Farbintensität im Mittel von 36 Digits (von minimal 35 bis maximal 37).

Je größer die Anzahl fluoreszierender Partikel einer bestimmten Größe im Polymergranulat ist, desto größer ist die Wahrscheinlichkeit Fließstörungen am fertigen Spritzgusskörper zu erhalten. Damit erhöht sich die Ausschussrate des jeweiligen Produkts. Überraschenderweise wurde gefunden, dass nicht alle fluoreszierenden Teilchen zu Fließstörungen führen, sondern nur Partikel einer bestimmten Größe. Aus optischen Disks wurden fluoreszierende Partikel, die unterhalb der Fließstörungen lagen, herauspräpariert (Mikrotom) und in der Schmelze wie oben beschrieben relaxiert. Es wurden lediglich kugelförmige Teilchen mit einem Durchmesser von 10 µm ermittelt. Dieser Befund war nicht nahe liegend.

Es bestand daher die Aufgabe ein hochreines Substratmaterial, vorzugsweise aus Polycarbonat, welches insbesondere für die Herstellung von Datenträgern, insbesondere solchen Datenträgern, welche mit blauen Laserlicht ausgelesen werden, sowie für die Herstellung von hochwertigen optischen Spritzgusskörpern geeignet ist, bereitzustellen.

Zur Lösung dieser Aufgabe mussten die für den Spritzgussprozess kritischen Teilchen identifiziert und ein Verfahren zur Detektion dieser Partikel zur Verfügung gestellt werden, um so die für einen qualitativ hochwertigen Spritzgusskörper kritische Größenverteilung an fluoreszierenden Partikeln zu ermitteln. Es konnte so das gewünschte Material zur Verfügung gestellt werden. Dieses material eignet sich insbesondere zur Herstellung von optischen Trägern wie CDs und DVDs, bei deren Herstellung kurze Zykluszeiten von beispielsweise kleiner 4s, bevorzugt kleiner 3s, und besonders bevorzugt von 0,3 bis 1,5 s wichtig sind.

Gegenstand der Erfindung ist daher ein Substratmaterial, bevorzugt aus Polycarbonat, welches eine bestimmte Größenverteilung an fluoreszierenden Teilchen mit Teilchendurchmesser pro Mengeneinheit Substratmaterial aufweist. Gegenstand der Erfindung ist daher ein Polycarbonat bzw. ein Substratmaterial, dadurch gekennzeichnet, dass die Verteilung des Teilchendurchmessers von fluoreszierenden Partikeln ausgewertet nach Lösen von 50 g Polycarbonat in 700 ml Dichlormethan und Filtration durch einen Teflonfilter mit 5 µm Porengröße bei einer Anregungswellenlänge von 400 - 440 nm und einer 50-fachen Gesamtvergrößerung und einer Belichtungszeit von 40 ms im Polycarbonat wie folgt ist (count pro Gramm Polycarbonat):
8.0 - ≤10 µm: 0.02 - 2.4 counts/g, bevorzugt 0.02 - 1.2 counts/g
>10 - ≤20 µm: 0.02 - 2.4 counts/g, bevorzugt 0.02 - 1.6 counts/g
>20 - ≤30 µm: 0.02 - 1.0 counts/g, bevorzugt 0.02 - 0.7 counts/g
>30 - ≤40 µm 0.02 - 0.6 counts/g, bevorzugt 0.02 - 0.4 counts/g
>40 - ≤50 µm: 0.02 - 0.5 counts/g, bevorzugt 0.02 - 0.3 counts/g
>50 - ≤80 µm: 0.02 - 0.4 counts/g, bevorzugt 0.02 - 0.2 counts/g
>80 - ≤200 µm: 0.02 - 0.4 counts/g, bevorzugt 0.02 - 0.2 counts/g
>200 - ≤400.0 µm: 0.02 - 0.2 counts/g, bevorzugt 0.02 - 0.1 counts/g

Hierbei wird ein auf dem Teflonfilter zusammenhängender fluoreszierender Bereich bei den oben angegebenen Bedingungen (Wellenlänge, Gesamtvergrößerung, Belichtungszeit) automatisch detektiert und als 1 "count" gezählt. Die einzelnen fluoreszierenden Partikel, die sich auf dem Teflonfilter befinden, werden ausgezählt. Die Gesamtzahl der fluoreszierenden Partikel wird durch die Masse der im jeweiligen Ansatz eingewogenen Polycarbonatschmelze dividiert und man erhält die Partikelanzahl (fluoreszierend) bezogen auf 1 Gramm Polycarbonat (counts/g).

Gegenstand der Erfindung ist weiterhin der aus dem Polycarbonat hergestellte Träger für eine optische Scheibe (bzw. Platte), dadurch gekennzeichnet, dass der Träger aus dem o. g. Polycarbonat hergestellt ist.

Aufgrund der rheologischen Eigenschaften dieser transparenten, fluoreszierenden Teilchen lassen sie sich nur schwer aus dem Substratmaterial abtrennen. Da diese Teilchen nicht hart sondern viskos sind, werden sie z. B. bei hohen Drücken oder hohen Temperaturen, bei denen die Polymermatrix als Schmelze vorliegt, von herkömmlichen Filtern nicht oder nur unzureichend zurückgehalten. Dies gilt z. B. für herkömmliche dünnlagige Metallsiebe. Dies gilt auch, wenn die Porengröße des Filters deutlich kleiner als der mittlere Durchmesser der fluoreszierenden Partikel ist. Bei höheren Drücken und/oder höheren Temperaturen nimmt die Verformbarkeit der Partikel zu, so dass diese Partikel herkömmliche Filtergewebe mit kleinen Porendurchmessern einfach durchdringen können.

Um Polycarbonate mit der erfindungsgemäßen Teilchengrößen-Verteilung zu erhalten, können bestimmte Filtrationsmedien genutzt werden, um Partikel von kritischer Größe, d.h. außerhalb des erfindungsgemäßen Bereiches zurückzuhalten. Dabei müssen verschiedene Parameter wie Druck, Konzentration und Temperatur so eingestellt werden, dass mit Hilfe der oben beschriebenen Methode sichergestellt ist, dass die Partikelzahlen im erfindungsgemäßen Bereich liegen. Ferner können während der Herstellung des erfindungsgemäßen Substratmaterials bestimmte Verfahrensparameter eingestellt werden, um die Entstehung fluoreszierender Partikel von kritischer Größe zu vermeiden. Dabei können z. B. schmelzeführende Leitungen aus herkömmlichen Stahl durch Rohre aus speziellen Legierungen wie z. B. Hastelloy ersetzt werden. Ferner kann dafür gesorgt werden, dass der Gradient zwischen der Rohr- bzw. Reaktortemperatur und der Temperatur in der Polymerschmelze nicht zu groß wird. Dies wird unten näher erläutert.

Diese Maßnahmen oder Kombinationen dieser Maßnahmen dienen dazu, dass erfindungsgemäße Substratmaterial zu erhalten.

Die vorliegende Erfindung ist also darin begründet, dass erfindungsgemäße Polycarbonate mit den erfindungsgemäßen Eigenschaften bereitstellt werden und für den Zweck verwendet werden, die erfindungsgemäßen Spritzgusskörper daraus herzustellen.

Die Herstellung der erfindungsgemäßen Polycarbonate kann sowohl nach dem Schmelzepolymerisations- als auch nach dem Phasengrenzflächenverfahren erfolgen. Das Phasengrenzflächenverfahren und das Schmelzepolymerisationsverfahren sind in der Literatur vielfach beschrieben. Beispielhaft sei auf H. Schnell, Chemistry and Physics of Polycarbonates, Polymer Reviews, Vol. 9, Interscience Publishers, New York 1964 S. 33 ff., auf Polymer Reviews, Vol. 10, "Condensation Polymers by Interfacial and Solution Methods", Paul W. Morgan, Interscience Publishers, New York 1965, Kap. VIII, S. 325, auf Dres. U. Grigo, K. Kircher und P. R- Müller "Polycarbonate" in BeckerBraun, Kunststoff-Handbuch, Band 3/1, Polycarbonate, Polyacetale, Polyester, Celluloseester, Carl Hanser Verlag München, Wien 1992, S. 118-145 sowie auf EP-A 0 517 044 bezüglich des Phasengrenzflächenverfahren verwiesen.

Gemäß diesem Verfahren erfolgt die Phosgenierung eines in wässrig-alkalischer Lösung (oder Suspension) vorgelegten Dinatriumsalzes eines Bisphenols (oder eines Gemisches verschiedener Bisphenole) in Gegenwart eines inerten organischen Lösungsmittels oder Lösungsmittelgemisches, welches eine zweite Phase ausbildet. Die entstehenden, hauptsächlich in der organischen Phase vorliegenden, Oligocarbonate werden mit Hilfe geeigneter Katalysatoren zu hochmolekularen, in der organischen Phase gelösten, Polycarbonaten aufkondensiert. Die organische Phase wird schließlich abgetrennt und das Polycarbonat durch verschiedene Aufarbeitungsschritte daraus isoliert.

Für die Herstellung der erfindungsgemäß zu verwendenden Polycarbonate geeignete Diphenole sind beispielsweise Hydrochinon, Resorcin, Dihydroxydiphenyl, Bis-(hydroxyphenyl)-alkane, Bis-(hydroxy-phenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren alkylierte, kernalkylierte und kernhalogenierte Verbindungen.

Bevorzugte Diphenole sind 4,4'-Dihydroxydiphenyl, 2,2-Bis-(4-hydroxyphenyl)-1-phenyl-propan, 1,1-Bis-(4-hydroxyphenyl)-phenyl-ethan, 2,2-Bis-(4-hydroxyphenyl)propan, 2,4-Bis-(4-hydroxy-phenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1-Bis-(4-hydroxyphenyl)-cyclohexan und 1,1-Bis-(4-hydroxyphenyl)-3,3,5-trimethylcyclohexan (Bisphenol TMC) sowie deren Mischungen.

Besonders bevorzugte Diphenole sind 4,4'-Dihydroxydiphenyl, 1,1-Bis-(4-hydroxyphenyl)-phenyl-ethan, 2,2-Bis-(4-hydroxyphenyl)-propan, 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC) sowie deren Mischungen.

Diese und weitere geeignete Diphenole sind z. B. in den US-A -PS 2 999 835, 3 148 172, 2 991 273, 3 271 367, 4 982 014 und 2 999 846, in den deutschen Offenlegungsschriften 1 570 703, 2 063 050, 2 036 052, 2 211 956 und 3 832 396, der französischen Patentschrift 1 561 518, in der Monographie "H. Schnell, Chemistry and Physics of Polycarbonates, Interscience Publishers, New York 1964, S. 28ff; S.102ff", und in "D.G. Legrand, J.T. Bendler, Handbook of Polycarbonate Science and Technology, Marcel Dekker New York 2000, S. 72ff." beschrieben

Im Falle der Homopolycarbonate wird nur ein Diphenol eingesetzt, im Falle der Copolycarbonate werden mehrere Diphenole eingesetzt, wobei selbstverständlich die verwendeten Bisphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Die zur Regelung des Molekulargewichtes benötigten monofunktionellen Kettenabbrecher, wie Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern, werden entweder mit dem Bisphenolat bzw. den Bisphenolaten der Reaktion zugeführt oder aber zu jedem beliebigen Zeitpunkt der Synthese zugesetzt, solange im Reaktionsgemisch noch Phosgen oder Chlorkohlensäureendgruppen vorhanden sind bzw. im Falle der Säurechloride und Chlorkohlensäureester als Kettenabbrecher solange genügend phenolische Endgruppen des sich bildenden Polymers zur Verfügung stehen. Vorzugsweise werden der oder die Kettenabbrecher jedoch nach der Phosgenierung an einem Ort oder zu einem Zeitpunkt zugegeben, wenn kein Phosgen mehr vorliegt, aber der Katalysator noch nicht dosiert wurde, bzw. sie werden vor dem Katalysator, mit dem Katalysator zusammen oder parallel dazu dosiert.

In der gleichen Weise werden eventuell zu verwendende Verzweiger oder Verzweigermischungen der Synthese zugesetzt, üblicherweise jedoch vor den Kettenabbrechern. Üblicherweise werden Trisphenole, Quarterphenole oder Säurechloride von Tri- oder Tetracarbonsäuren verwendet, oder auch Gemische der Polyphenole oder der Säurechloride.

Einige der verwendbaren Verbindungen mit drei oder mehr als drei phenolischen Hydroxylgruppen sind beispielsweise Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl]-propan, 2,4-Bis-(4-hydroxyphenyl-isopropyl)-phenol, Tetra-(4-hydroxyphenyl)-methan.

Einige der sonstigen trifunktionellen Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Bevorzugte Verzweiger sind 3,3-Bis-(3-methyl-4-hydroxyphenyl)-2-oxo-2,3-dihydroindol und 1, 1, 1-Tri-(4-hydroxyphenyl)-ethan.

Die in der Phasengrenzflächensynthese verwendeten Katalysatoren sind tert. Amine, insbesondere Triethylamin, Tributylamin, Trioctylamin, N-Ethylpiperidin, N-Methylpiperidin, N-i/n-Propylpiperidin; quartäre Ammoniumsalze wie Tetrabutylammonium- / Tributylbenzylammonium- / Tetraethylammonium- hydroxid / -chlorid / -bromid / -hydrogen-sulfat / -tetrafluoroborat; sowie die den Ammoniumverbindungen entsprechenden Phosphoniumverbindungen. Diese Verbindungen sind als typische Phasengrenzflächen-Katalysatoren in der Literatur beschrieben, kommerziell erhältlich und dem Fachmann geläufig. Die Katalysatoren können einzeln, im Gemisch oder auch neben- und nacheinander der Synthese zugesetzt werden, gegebenenfalls auch vor der Phosgenierung, bevorzugt sind jedoch Dosierungen nach der Phosgeneintragung, es sei denn, es wird eine Oniumverbindung oder Gemische aus Oniumverbindungen als Katalysatoren verwendet, dann ist eine Zugabe vor der Phosgendosierung bevorzugt. Die Dosierung des Katalysators oder der Katalysatoren kann in Substanz, in einem inerten Lösungsmittel, vorzugsweise dem der Polycarbonatsynthese, oder auch als wässrige Lösung, im Falle der tert. Amine dann als deren Ammoniumsalze mit Säuren, bevorzugt Mineralsäuren, insbesondere Salzsäure, erfolgen. Bei Verwendung mehrerer Katalysatoren oder der Dosierung von Teilmengen der Katalysatorgesamtmenge können natürlich auch unterschiedliche Dosierungsweisen an verschiedenen Orten oder zu verschiedenen Zeiten vorgenommen werden. Die Gesamtmenge der verwendeten Katalysatoren liegt zwischen 0,001 bis 10 Mol % bezogen auf Mole eingesetzte Bisphenole, bevorzugt 0,01 bis 8 Mol %, besonders bevorzugt 0,05 bis 5 Mol %.

Daneben ist die Herstellung von Polycarbonaten auch aus Diarylcarbonaten und Diphenolen nach dem bekannten Polycarbonatverfahren in der Schmelze, dem so genannten Schmelzeumesterungsverfahren, möglich, das z. B. in WO-A 01/05866 und WO-A 01/05867 beschrieben ist. Daneben werden Umesterungsverfahren (Acetatverfahren und Phenylesterverfahren) beispielsweise in den US-A 34 94 885, 43 86 186, 46 61 580, 46 80 371 und 46 80 372, in den EP-A 26 120, 26 121, 26 684, 28 030, 39 845, 91 602, 97 970, 79 075, 14 68 87, 15 61 03, 23 49 13 und 24 03 01 sowie in den DE-A 14 95 626 und 22 32 977 beschrieben.

Für die Herstellung des erfindungsgemäßen Substratmaterials ist insbesondere das kontinuierliche Herstellverfahren für Polycarbonat nach dem Phasengrenzflächenverfahren geeignet. Besonders bevorzugt ist eine Kontireaktion, die einen Umpumpreaktor als Phosgenierreaktor und nachgeschaltete Rohrreaktoren verwendet.

Für die Herstellung des erfindungsgemäßen Substratmaterials ist insbesondere auch die anschließende Art und Weise der Isolierung des Polycarbonats von Bedeutung.

Bevorzugt erfolgt die Eindampfung der Polycarbonatlösung aus einem Chlorbenzol/Dichlormethan-Gemisch, mit einem Chlorbenzolgehalt von 20 - 70 Gew-% bevorzugt 40 - 60 Gew-%. Insbesondere geeignet ist die Eindampfung der Polycarbonatlösung in einem thermischen Verfahren, dem so genannten Flash-Prozess. Dabei erfolgt die Konzentrierung der Polymerlösung und eventuell auch die Isolierung des Polymeren durch Abdestillation des Lösungsmittels indem durch Überhitzung und Entspannung das Lösungsmittel entfernt wird. Dieser Prozess ist dem Fachmann geläufig, siehe auch "Thermische Trennverfahren", VCH Verlagsanstalt 1988, S. 114. Bei dem bekannten Flashverfahren werden Polymerlösungen wiederholt unter leichtem Überdruck auf Temperaturen oberhalb des Siedepunktes unter Normaldruck erhitzt und diese, bezüglich des Normaldruckes, überhitzten Lösungen anschließend in ein Gefäß mit niedrigerem Druck, z. B. Normaldruck, entspannt. Es kann dabei von Vorteil sein, die Aufkonzentrationsstufen, oder anders ausgedrückt die Temperaturstufen der Überhitzung nicht zu groß werden zu lassen sondern lieber ein zwei- bis vierstufiges Verfahren zu wählen. Besonders bevorzugt ist ein dreistufiges Eindampfverfahren mit nachfolgender Isolierung des Polycarbonats über einen Ausdampfextruder. Zur Herstellung des erfindungsgemäßen Substratmaterials kann es vorteilhaft sein, wenn in einem mehrstufigen Eindampfprozess gearbeitet wird, wobei die Temperaturdifferenzen zwischen den Eindampfstufen nicht mehr als 90°C, besonders bevorzugt nicht mehr als 60°C betragen. Weiterhin ist es wichtig, für eine effektive Aufkonzentrierung zu sorgen, um im abschließenden Eindampfschritt, bevorzugt ist ein Ausdampfextruder, ein Material von hoher Qualität herzustellen. Dabei ist es bevorzugt wenn in der ersten Eindampfstufe die Temperaturen 70 - 80°C bei 0.35 - 0.45 bar Überdruck, in der zweiten Eindampfstufe die Temperaturen 125 - 135°C bei 0.75 - 0.85 bar Überdruck und in der dritten Eindampfstufe 175 - 185°C bei 1.85 - 1.95 bar Überdruck betragen.

Aus den so erhaltenen hochkonzentrierten Polymerschmelzen können die Reste des Lösungsmittels entweder direkt aus der Schmelze mit Ausdampfextrudem (BE-A 866 991, EP-A 0 411 510, US-A 4 980 105, DE-A 33 32 065), Dünnschichtverdampfern (EP-A 0 267 025), Fallfilmverdampfern, Strangverdampfem oder durch Friktionskompaktierung (EP-A 0 460 450), gegebenenfalls auch unter Zusatz eines Schleppmittels, wie Stickstoff oder Kohlendioxid oder unter Verwendung von Vakuum (EP-A 0 039 96, EP-A 0 256 003, US-A 4 423 207), entfernt werden, alternativ auch durch anschließende Kristallisation (DE-A 34 29 960) und Ausheizen der Reste des Lösungsmittels in der festen Phase ( US-A 3 986 269, DE-A 20 53 876). Dabei ist die Isolierung mittels Ausdampfextrudern bevorzugt.

Granulate erhält man, wenn möglich, durch direktes Abspinnen der Schmelze und anschließender Granulierung oder aber durch Verwendung von Austragsextrudern von denen in Luft oder unter Flüssigkeit, meist Wasser, abgesponnen wird. Werden Extruder benutzt, so kann man der Schmelze, vor diesem Extruder, gegebenenfalls unter Einsatz von statischen Mischern oder durch Seitenextruder im Extruder, Additive zusetzen.

Die erfindungsgemäßen Polycarbonate werden ferner erhalten, in dem man den Produktionsprozess wie oben angegeben steuert und mit einer gekoppelten automatisierten Auswertung prüft, ob das Polycarbonat die erfindungsgemäße Teilchengrößenverteilung an fluoreszierenden Partikeln aufweist. Alternativ kann dem Produktionsprozess eine Filtration folgen mit einer gekoppelten automatisierten Auswertung zur Qualitätskontrolle des Polycarbonats.

Ein geeignetes Filtrationsverfahren von Polycarbonat ist beispielsweise dadurch gekennzeichnet, dass die Schmelze von Polycarbonat zumindest ein Metallvlies bestehend aus mehr als einer Schicht dünner Metalldrähte filtriert wird. Die Drähte sind dabei zu Lagen gestapelt und können gegebenenfalls durch dickere Stützdrähte stabilisiert werden. Das Metallvlies besteht aus Drähten von z. B. V4A Stahl, z. B. mit der Werkstoff-Nr. 1.4571. Die Stützdrähte können beispielsweise aus V4A 1.4571 oder V2A 1.4541 oder Stahl mit der Werkstoff-Nr. 1.4310 bestehen. Das Vlies besitzt eine Dicke von 5 mm bis 0,1 mm, bevorzugt 1 mm bis 0,2 mm. Das Vlies enthält unterschiedliche dichte Lagen. Die dichteste Lage ist bevorzugt 0,05 bis 0,2 mm dick. Weiterhin bevorzugt besteht die dichteste Lage aus dünnen Drähten mit einem Durchmesser von 1 - 20 µm, bevorzugt 2 - 10 µm, insbesondere bevorzugt 2 - 7 µm. Die Porengröße in der weniger dichten Lage beträgt bevorzugt 20 - 60 µm, die in der dichtesten Lage bevorzugt 1 - 10 µm. Diese Drähte können glatt oder oberflächenstrukturiert sein, was durch spezielle Behandlungsmethoden wie durch Behandlung mit Säure, wie z.B. Salpetersäure oder Citronensäure oder durch Elektropolieren erreicht werden kann.

Das Filtervlies bzw. alle Metalloberflächen des Filters werden thermisch vorbehandelt. Die thermische Vorbehandlung kann in Schutzgasatmosphäre wie Stickstoff oder Argon oder in oxidativer Atmosphäre wie Luft erfolgen, bevorzugt in oxidativer Atmosphäre.

Die thermische Vorbehandlung wird im Allgemeinen bei Temperaturen von 300 bis 1200°C je nach Art der Temperung bzw. der entsprechenden Tempertemperatur durchgeführt. Die Dauer der thermischen Vorbehandlung beträgt im Allgemeinen 1 Minute bis 48 Stunden, bevorzugt 2 Minuten bis 30 Stunden und insbesondere 10 Minuten bis 24 Stunden und ist abhängig von der Art der Temperung.

Die thermische Vorbehandlung kann dabei durch Glühen der Metalloberfläche in einer Flamme, durch Temperung in einem Muffelofen oder durch Temperung in einem Umluftofen erfolgen.

Beim Glühen der Metalloberflächen in einer Glasflamme liegen die Temperaturen üblicherweise zwischen 600 und 1500°C, bevorzugt zwischen 800 und 1200°C, bei der Temperung im Muffelofen zwischen 300 und 1000°C, bevorzugt zwischen 600 und 900°C und bei der Temperung im Umluftofen bei 200 bis 500°C, bevorzugt bei 300 bis 400°C.

Die Dauer der thermischen Vorbehandlung bei Temperaturen oberhalb von 600°C beträgt im Allgemeinen 1 Minute bis 1 Stunde, im Temperaturbereich von 300 bis 700°C in der Regel 30 Minuten bis 48 Stunden, bevorzugt 30 Minuten bis 24 Stunden, bei Temperaturen von 300 bis 500°C im Allgemeinen 1 bis 48 Stunden, bevorzugt 2 bis 24 Stunden.

Das Ausflammen der durch Glühen behandelten Metallteile erfolgt in einem Sand- oder Wirbelbett.

Die Erfindung wird durch die folgenden Beispiele erläutert, ist jedoch nicht auf diese Beispiele eingeschränkt.

### Beispiele

### Methode zur Bestimmung des Gehalts an fluoreszierenden Partikeln:

Die Analyse des Gehalts an fluoreszierenden Partikeln erfolgt durch Filtration der betreffenden Polycarbonatprobe (50 g), gelöst in Dichlormethan (LiChrosolv; Merck:1.06044 K33506244 430) (700 ml) durch einen Teflon-Filtermembran ( Fa. Bohlender GmbH, D-97847 Grünsfeld) mit 5 µm Porendurchmesser und einer Filtermembranstärke von 1mm. Die Filterscheiben werden im Vakuum getrocknet und vor Umgebungsstaub durch eine Abdeckung geschützt. Nach Filtration wird die Filteroberfläche mittels eines Fluoreszenzmikroskops Axioplan 2 der Firma Zeiss AG, Deutschland, untersucht (gescannt). Es wird mit einer Anregungswellenlänge von 400 - 440 nm, einer Belichtungszeit von 40 ms pro Scan und einer 50-fachen Gesamtvergrößerung gearbeitet. Die fluoreszierenden Partikel werden detektiert und die Daten über eine Bildverarbeitungssoftware (KS 300 3.0 der Firma Zeiss AG) ausgewertet. Es werden nur Partikel mit einer charakteristischen Farbe gezählt, d.h. andere Partikel, wie z. B. Staub werden nicht berücksichtigt. Die Farbparameter zur Erkennung der fluoreszierenden Partikel werden so eingestellt, dass sie den Parametern der bei Fließstörungen in optischen Disks gefundenen Teilchen gleichen. Die Abrasterung der Oberfläche des Filters erfolgt automatisch über einen computergesteuerten Objekttisch (Fa. Zeiss AG).

Zur Herstellung der erfindungsgemäßen Formteile - hier als Beispiel eine optische Disk - wird eine Netstal Discjet verwendet. Es werden folgende Spritzgussparameter und Bedingungen eingestellt:
Maschine: Netstal Discjet HybridMatritze: Audio-Stamper
Zykluszeit: 3,2 - 3,6 s (in den genannten Beispielen: 3,4 s)
Massetemperatur: 330 °C
Substratdimensionen: Audio-CD
Werkzeugtemperatur matrizenseitig: 42 °C
Werkzeugtemperatur spiegelseitig: 43 °C

Die Disks werden vor Untersuchung auf Fließstörungen mit einer reflektierenden Schicht versehen (Vakuumbeschichtung mit Aluminium). Die Disks werden über einen Scanner auf Fließstörungen hin überprüft. Dabei ist der Scanner so eingestellt, dass nur Fließstörungen erfasst werden, d.h. Fehler wie Flecken, Kratzer und Fremdkörpereinschlüsse werden nicht erfasst. Dabei werden die aufgenommenen Informationen über eine Bildverarbeitungssoftware ausgewertet; Disks mit Defekten werden aussortiert. Als so genannte Fließstörungen werden nur radial verlaufende (d.h. in Fließrichtung verlaufende) strichförmige Defekte ohne Fremdkörpereinschlüsse anerkannt. Um dies sicherzustellen, werden die mit einem Defekt aussortierten Disks noch einmal visuell kontrolliert.

Als Scanner und als Auswertungssoftware werden Produkte der Firma Dr. Schenk GmbH, 82152 Planegg, Deutschland verwendet.

Das in den folgenden Beispielen verwendete Polycarbonat (Bisphenol A als Diphenol, p-tert.-Butylphenol als Kettenabbrecher) wird nach dem üblichen Phasengrenzflächenverfahren hergestellt. Dazu werden ein Umpumpreaktor und zwei anschließende Verweilreaktoren verwendet. In den Umpumpreaktor werden die Bisphenolatlösung (Bisphenol A), das Lösungsmittel und das Phosgen dosiert und zur Reaktion gebracht, wobei die erste Natronlaugenmenge ebenfalls in den Umpumpreaktor dosiert wird. Vor dem ersten Verweilreaktor werden die zweite Natronlaugenmenge und die Kettenabbrecher-Lösung (p-tert.-Butylphenol in Methylenchlorid) dosiert. Vor dem zweiten Verweilreaktor wird die Katalysator-Lösung (N-Ethylpiperidin als Katalysator) dosiert.

Nach dem zweiten Verweilreaktor werden die Phasen getrennt, die organische Phase mit ca. 1 gew.-%iger Salzsäure und 5 mal mit Wasser gewaschen.

Das so erhaltene Polycarbonat hat ein mittleres Molekulargewicht (Gewichtsmittel) von 17000 bis 18000 g/mol (GPC-Messung).

### Beispiel 1 (erfindungsgemäßes Beispiel)

Das wie oben beschrieben hergestellte Polycarbonat wird in einen Zwischentank abgelassen. Die Konzentration des Polycarbonats gelöst in einem Gemisch aus Dichlormethan und Chlorbenzol (1:1 Gew-%) beträgt ca. 16.5 Gew-%. Diese Lösung wird über 2 Eindampfstufen bei einem Temperaturgradienten von 60 bis 220°C, einem Druck von 1.1 bis 2.3 bar bei einer mittleren Verweilzeit von 30 Minuten auf ca. 75 % eingeengt. Diese Lösung wird über eine 3. Eindampfstufe, eine Flashstufe mit Abscheider, weiterbehandelt. Das Polycarbonat wird über eine beheizte Austragspumpe gefördert und einer Granulierung zugeführt. Die Eindampfstufen bestehen jeweils aus 4 Vorwärmern mit Druckhalteventil, einem Wendelrohrverdampfer und einem Abscheider mit Austragspumpe. Die Polycarbonatlösung wird im Vorwärmer aufgeheizt - das Druckhalteventil sorgt dafür, dass es im Vorwärmer nicht zum Sieden kommt. Im Wendelrohrverdampfer und Abscheider dampft (flasht) das Lösungsmittel aus. Im ersten Eindampfschritt wird die Polycarbonatlösung eingeengt von 16.5 auf 30 - 35 %. In der zweiten Eindampfstufe wird weiterhin aufkonzentriert bis eine Konzentration von 70 - 75 % erreicht wird. In einem dritten Eindampfschritt, eine Flashstufe mit Abscheider und Austragspumpe, wird die PC-Lösung bei einer mittleren Temperatur von 250°C und einen Abscheiderdruck von 100 mbar weiter aufkonzentriert. Der Stand in Abscheider beträgt ~10 % - dies entspricht einer Verweilzeit von ca. 0.5 Minuten. Die Austragspumpe fördert die Schmelze zu einem Granulator. Das Polycarbonat hat ein mittleres Molekulargewicht (Gewichtsmittel) von 17000 bis 18000 g/mol.

An dem so erhaltenen PC-Granulat wird die Teilchengrößenverteilung der fluoreszierenden Partikel nach der oben angegebenen Methode bestimmt.

Das Ergebnis der Untersuchung ist in Tabelle 1 gezeigt.

### Beispiel 2 (Vergleichsbeispiel)

Das Polycarbonat mit einem Molekulargewicht (Gewichtsmittel) von M_{w} = 17.000-18.000 g/mol wird wie oben beschrieben hergestellt. Die Aufarbeitung und Isolierung des Polycarbonats erfolgt im Prinzip wie in Beispiel 1 beschrieben. Allerdings beträgt die Temperatur in der dritten Eindampfstufe 280°C. Der Stand im Abscheider vor der Austragspumpe beträgt > 25 % und entspricht einer mittleren Verweilzeit von 6.5 Minuten.

An dem so erhaltenen PC-Granulat wird die Teilchengrößenverteilung der fluoreszierenden Partikel nach der oben angegebenen Methode bestimmt. Das Ergebnis der Untersuchung ist in Tabelle 1 gezeigt.

### Aus dem Polycarbonat der Beispiele 1 und 2 werden wie folge Disks hergestellte:

30 kg des erhaltenen Granulats gemäß Beispiels 1 bzw. 2 werden für 6 Stunden getrocknet und dann über eine Netstal Hybrid Spritzgussmaschine bei einer Zykluszeit von 3,4 Sekunden unter den oben angegebenen Parametern zu Disks verarbeitet. Als Matritze wird ein Audiostamper verwendet.

Die Ergebnisse sind in Tabelle 1 zusammengefasst.

**Tabelle 1**

| **Größenklasse Fluoreszierende Partikel** | **Beispiel 1 (Erfindungsgemäß) Anzahl fluoreszierender Partikel** | **Beispiel 2 (Vergleich) Anzahl fluoreszierender Partikel** |
|---|---|---|
| 8.0 - 10.0 µm | 1.06 counts/g | 5.68 counts/g |
| 10.1 - 20.0 µm | 1.46 counts/g | 6.94 counts/g |
| 20.1 - 30.0 µm | 0.68 counts/g | 3.04 |
| 30.1 - 40.0 µm | 0.34 | 1.76 |
| 40.1 - 50.0 µm | 0.24 | 1.22 |
| 50.1 - 80.0 µm | 0.16 | 1.74 |
| 80.1 - 200.0 µm | 0.2 | 1.72 |
| 200.1 - 400.0 µm | 0.06 | 0.36 |
| **Gesamt-Count** | 4.20 | 22.46 |
| **Fluores. Fließstörung am Spritzgusskörper (CD)** | 3.33 % | 63.50 % |

## Patentansprüche

1. Polycarbonat, **gekennzeichnet, durch** folgende Verteilung der Teilchendurchmesser von enthaltenen fluoreszierenden Partikeln
8.0 - ≤10 µm: 0.02 - 2.4 counts/g,
>10 - ≤20 µm: 0.02 - 2.4 counts/g,
>20 - ≤30 µm: 0.02 - 1.0 counts/g,
>30 - ≤40 µm: 0.02 - 0.6 counts/g,
>40 - ≤50 µm: 0.02 - 0.5 counts/g,
>50 - ≤80 µm: 0.02 - 0.4 counts/g,
>80 - ≤200 µm: 0.02 - 0.4 counts/g,
>200 - ≤400.0 µm: 0.02 - 0.2 counts/g,
bestimmt nach Lösen von 50 g Polycarbonat in 700 ml Dichlormethan und Filtration **durch** einen Teflonfilter mit 5 µm Porengröße bei einer Anregungswellenlänge von 400 - 440 nm und einer 50-fachen Gesamtvergrößerung und einer Belichtungszeit von 40 ms pro Scan.

2. Polycarbonate gemäß Anspruch 1, **gekennzeichnet durch** eine Teilchengrößenverteilung an enthaltenen fluoreszierenden Partikeln
8.0 - ≤10 µm: > 0.02 - 1.2 counts/g
>10 - ≤20 µm: > 0.02 - 1.6 counts/g
>20 - ≤30 µm: > 0.02 - 0.7 counts/g
>30 - ≤40 µm: > 0.02 - 0.4 counts/g
>40 - ≤50 µm: > 0.02 - 0.3 counts/g
>50 - ≤80 µm: > 0.02 - 0.2 counts/g
>80 - ≤200 > 0.02 - 0.2 counts/g
>200 - ≤400 µm: > 0.02 - 0.1 counts/g.

3. Substratmaterial aus Polycarbonat **dadurch gekennzeichnet, dass** das Substratmaterial eine Teilchengrößenverteilung an enthaltenen Fluoreszenzpartikeln gemäß Anspruch 1 oder 2 aufweist

4. Verwendung von Polycarbonat gemäß Anspruch 1 oder 2 als Substratmaterial zur Herstellung von Formteilen und Extrudaten.

5. Formteile und Extrudate aus Polycarbonat oder Substratmaterialien nach Anspruch 1 oder 2.

6. Formteil nach Anspruch 5, wobei das Formteil eine optische Disk ist.

7. Formteil nach Anspruch 5, wobei das Formteil eine Streuscheibe ist.

## Claims

1. Polycarbonate **characterized by** the following distribution of the particle diameters of fluorescent particles present:
8.0 - ≤ 10 µm: 0.02 - 2.4 counts/g,
> 10 - ≤ 20 µm: 0.02 - 2.4 counts/g,
> 20 - ≤ 30 µm: 0.02 - 1.0 counts/g,
> 30 - ≤ 40 µm: 0.02 - 0.6 counts/g,
> 40 - ≤ 50 µm: 0.02 - 0.5 counts/g,
> 50 - ≤ 80 µm: 0.02 - 0.4 counts/g,
> 80 - ≤ 200 µm: 0.02 - 0.4 counts/g,
> 200 - ≤ 400 µm: 0.02 - 0.2 counts/g,
determined after dissolution of 50 g of polycarbonate in 700 ml of dichloromethane and filtration through a Teflon filter with 5 µm pore size with an excitation wavelength of 400-440 nm and with a total magnification of 50× and with an exposure time of 40 ms per scan.

2. Polycarbonates according to Claim 1, **characterized by** a particle size distribution of fluorescent particles present:
8.0 - ≤ 10 µm: 0.02 - 1.2 counts/g,
> 10 - ≤ 20 µm: 0.02 - 1.6 counts/g,
> 20 - ≤ 30 µm: 0.02 - 0.7 counts/g,
> 30 - ≤ 40 µm: 0.02 - 0.4 counts/g,
> 40 - ≤ 50 µm: 0.02 - 0.3 counts/g,
> 50 - ≤ 80 µm: 0.02 - 0.2 counts/g,
> 80 - ≤ 200 µm: 0.02 - 0.2 counts/g,
> 200 - ≤ 400 µm: 0.02 - 0.1 counts/g.

3. Substrate material made of polycarbonate, **characterized in that** the substrate material has a particle size distribution of fluorescent particles present according to Claim 1 or 2.

4. Use of polycarbonate according to Claim 1 or 2 as substrate material for the production of mouldings and extrudates.

5. Mouldings and extrudates made of polycarbonate or substrate materials according to Claim 1 or 2.

6. Moulding according to Claim 5, where the moulding is an optical disc.

7. Moulding according to Claim 5, where the moulding is a diffuser sheet.

## Revendications

1. Polycarbonate, **caractérisé par** la distribution suivante des diamètres de particules de particules fluorescentes contenues :
8,0 à ≤ 10 µm : 0,02 à 2,4 coups/g,
> 10 à ≤ 20 µm : 0,02 à 2,4 coups/g,
> 20 à ≤ 30 µm : 0,02 à 1,0 coup/g,
> 30 à ≤ 40 µm : 0,02 à 0,6 coup/g,
> 40 à ≤ 50 µm : 0,02 à 0,5 coup/g,
> 50 à ≤ 80 µm : 0,02 à 0,4 coup/g,
> 80 à ≤ 200 µm : 0,02 à 0,4 coup/g,
> 200 à ≤ 400 µm : 0,02 à 0,2 coup/g,
déterminée par dissolution de 50 g de polycarbonate dans 700 ml de dichlorométhane et filtration par un filtre en Téflon d'une taille de pores de 5 µm à une longueur d'onde d'excitation de 400 à 440 nm et à un agrandissement total de 50 fois et une durée d'éclairage de 40 ms par balayage.

2. Polycarbonates selon la revendication 1, **caractérisés par** une distribution des tailles de particules de particules fluorescentes contenues :
8,0 à ≤ 10 µm : > 0,02 à 1,2 coups/g,
> 10 à ≤ 20 µm : > 0,02 à 1,6 coups/g,
> 20 à ≤ 30 µm : > 0,02 à 0,7 coup/g,
> 30 à ≤ 40 µm : > 0,02 à 0,4 coup/g,
> 40 à ≤ 50 µm : > 0,02 à 0,3 coup/g,
> 50 à ≤ 80 µm : > 0,02 à 0,2 coup/g,
> 80 à ≤ 200 > 0,02 à 0,2 coup/g,
> 200 à ≤ 400 µm : > 0,02 à 0,1 coup/g.

3. Matériau substrat en polycarbonate, **caractérisé en ce que** le matériau substrat présente une distribution des tailles de particules de particules fluorescentes contenues selon la revendication 1 ou 2.

4. Utilisation d'un polycarbonate selon la revendication 1 ou 2 en tant que matériau substrat pour la fabrication de pièces moulées et d'extrudats.

5. Pièces moulées et extrudats en polycarbonate ou matériaux substrats selon la revendication 1 ou 2.

6. Pièce moulée selon la revendication 5, dans laquelle la pièce moulée est un disque optique.

7. Pièce moulée selon la revendication 5, dans laquelle la pièce moulée est un panneau diffusant.
